# FASCICULE DE BREVET EUROPEEN

(11) **EP 0 773 223 B1**
(45) Date de publication et mention de la délivrance du brevet: **17.01.2001**
(21) Numéro de dépôt: 96402366.7
(22) Date de dépôt: 07.11.1996
(51) Int. Cl.: C07D 498/04, C07D 495/04, C07D 495/14, A61K 31/535

(54) **Nouveaux composés tétracycliques de la 1,4-oxazine, leur procédé de préparation et les compositions pharmaceutiques qui les contiennent**
Tetrazyclisch 1,4-Oxazinverbindungen, Verfahren zu ihrer Herstellung und diese enthaltende pharmazeutische Zubereitungen
Tetracyclic 1,4-oxazine compounds, process for their preparation and pharmaceutical compositions containing them

(30) Priorité: 09.11.1995 FR 9513270
(43) Date de publication de la demande: 14.05.1997
(73) Titulaire: ADIR ET COMPAGNIE, 92415 Courbevoie Cédex (FR)
(72) Inventeur: Peglion, Jean-Louis, 78110 Le Vesinet (FR); Goument, Bertrand, 78220 Viroflay (FR); Harmange, Jean-Christophe, 78100 Saint Germain en Laye (FR); Millan, Mark, 78230 Le Pecq (FR); Audinot, Valérie, 78300 Poissy (FR)

(56) Documents cités:
- EP-A- 0 246 633
- WO-A-94/22495

## Description

La présente invention a pour objet de nouveaux composés tétracycliques de la 1,4-oxazine, leur procédé de préparation et les compositions pharmaceutiques qui les contiennent.

Elle concerne plus spécialement les composés tétracycliques de la 1,4-oxazine de formule **I** : 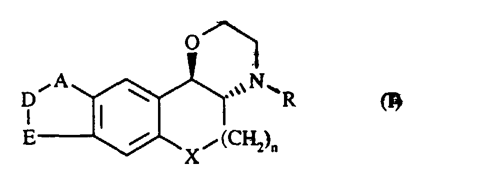 dans laquelle:
∗ **-A-D-E-** représente : 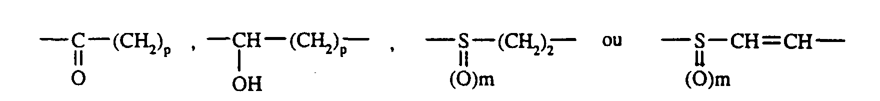 dans lesquels :
   . p représente 2 ou 3 et
   . m représente zéro, 1 ou 2 ;
∗ **X** représente :
   . un groupe CH₂ et de plus,
   . lorsque -A-D-E- représente 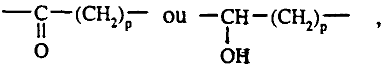 **X** peut aussi représenter un atome d'oxygène ;
∗ **n** représente :
   . zéro ou 1 lorsque X représente le groupe CH₂, et
   . uniquement 1 lorsque X représente un atome d'oxygène ;
∗ **R** représente :
   . un atome d'hydrogène ou
   . un radical (C₁-C₁₀)alkyle, (C₃-C₁₀)alcényle ou (C₃-C₁₀)alcynyle, chacun en chaîne droite ou ramifiée et chacun éventuellement substitué par un ou plusieurs radicaux cycloalkyles ayant de 3 à 8 atomes de carbone, ou par un radical aryle choisi parmi les radicaux phényle, thiényle et pyridyle chacun d'eux éventuellement substitué par un ou plusieurs substituants choisis parmi les atomes d'halogène, le radical hydroxy et les radicaux alkyle et alkoxy ayant chacun de 1 à 6 atomes de carbone en chaîne droite ou ramifiée.

Les composés de l'invention possèdent la jonction de cycle trans entre le cycle 1,4-oxazine et le cycle qui lui est adjacent.

La présence de carbones asymétriques implique que les composés de l'invention existent sous la forme de mélange racémique et d'isomères optiques également inclus dans la présente invention.

De plus, les composés de formule I peuvent former, avec des acides organiques ou inorganiques pharmaceutiquement acceptables, des sels d'addition acides physiologiquement tolérables qui font aussi partie de la présente invention.

L'état antérieur de la technique le plus proche de la présente invention est constitué par :
- les composés du benzopyrane de formule : 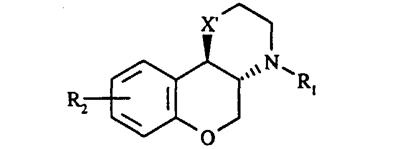 dans laquelle :
   . R₂ n'inclut ni ne suggère le groupe A-D-E précédemment défini, et
   . X' représente :
      un atome d'oxygène (cf. EP 0 246 633) ou
      un groupe CH₂ (cf. EP 0 161 218)
- ainsi que les composés de formule : 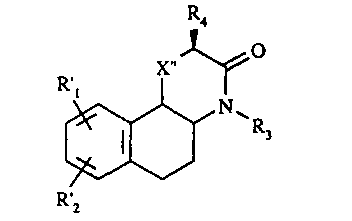 décrits dans la demande WO 93.24471.

Ces substances exercent leur action au niveau du récepteur dopaminergique D₂ auquel elles se lient très fortement. De ce fait, elles sont utilisables, lorsqu'elles sont des bloqueurs de ce récepteur D₂, dans le traitement de la schizophrénie et, lorsqu'elles sont des activateurs de ce même récepteur, dans le traitement de la maladie de Parkinson.

Toutefois, leur forte activité au niveau du récepteur D₂ fait que leur utilisation entraîne des effets secondaires gênants tels que dyskinésie tardive, hyperprolactinémie et aménorrhée dans le cas des agents bloqueurs, effets cardio-vasculaires et moteurs dans le cas des activateurs.

La découverte récente d'un nouveau récepteur à la dopamine, dénommé le récepteur D₃, dont la concentration est très importante au niveau du système limbique mais très faible dans le noyau nigrostrié et dans les cellules lactotrophes, encourage la recherche de nouveaux médicaments agissant au niveau du système dopaminergique mais ayant pour cible préférentielle le récepteur D₃ et de ce fait exempts des effets secondaires liés typiquement à l'activité sur le récepteur D₂ comme mentionné précédemment.

Des études réalisées in vitro (binding sur récepteurs clonés D₂ et D₃) avec les composés de la présente invention montrent que ceux-ci se comportent comme des ligands très affins au niveau du récepteur dopaminergique D₃ tout en ne présentant que peu d'affinité pour le récepteur dopaminergique D₂.

Cette sélectivité confère aux composés de la présente invention un intérêt tout particulier pour leur utilisation comme médicament agissant au niveau du système dopaminergique sans causer les effets indésirables des ligands D₂. Cette activité des composés de la présente invention a été également mise en évidence in vivo grâce au test de réversion de l'hypothermie induite par l'agoniste prototypique D₃ (7-OH-DPAT) selon la méthode de M.J. Millan, Eur. J. Pharmacol. (1994), 260, R₃-R₅.

Les composés de la présente invention se différencient donc des produits de l'état antérieur de la technique non seulement par leur structure chimique mais aussi par leur activité pharmacologique qui leur permet d'exercer un effet bénéfique lors de leur utilisation dans le traitement de la maladie de Parkinson [J. Neur. Transm. (1993), 94, 11-19], des troubles de la mémoire [Nature (1990), 347, 146-151], de l'abus de drogue [Science (1993), 260, 1814], de la dépression et des psychoses.

La présente invention a également pour objet le procédé de préparation des composés de formule I caractérisé en ce que :
∗ l'on fait réagir :
   . une amine primaire de formule II : 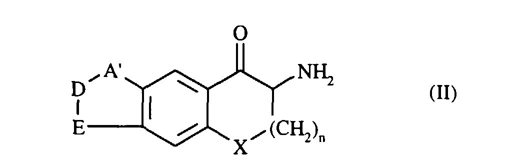 dans laquelle :
      X et n ont les significations précédemment définies et
      -A'-D-E- représente : -(CH₂)₃-, -(CH₂)₄-, -S-(CH₂)₂- ou -S-CH=CH-
   . avec un composé halogéné de formule III : 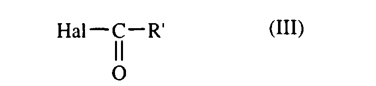 dans laquelle :
      Hal représente un atome de chlore ou de brome et
      R' représente :
         un radical cycloalkyle ayant de 3 à 8 atomes de carbone ;
         un radical aryle choisi parmi les radicaux phényle, thiényle et pyridyle chacun d'eux éventuellement substitué par un ou plusieurs substituants choisis parmi les atomes d'halogène, le radical hydroxy et les radicaux alkyle et alkoxy ayant chacun de 1 à 6 atomes de carbone en chaîne droite ou ramifiée, ou
         un radical (C₁-C₉)alkyle, (C₂-C₉)alcényle ou (C₂-C₉)alcynyle, chacun en chaîne droite ou ramifiée et chacun éventuellement substitué par un ou plusieurs radicaux cycloalkyles ayant de 3 à 8 atomes de carbone, ou par un radical aryle choisi parmi les radicaux phényle, thiényle et pyridyle chacun d'eux éventuellement substitué par un ou plusieurs substituants choisis parmi les atomes d'halogène, le radical hydroxy et les radicaux alkyle et alkoxy ayant chacun de 1 à 6 atomes de carbone en chaîne droite ou ramifiée,
∗ pour obtenir un composé de formule IV : 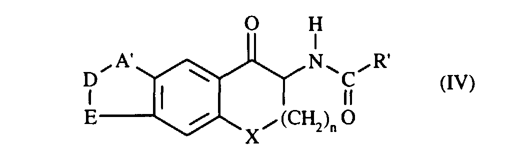 dans laquelle A'-D-E, X, n et R' ont les significations précédemment définies,
∗ lequel composé de formule IV est alors réduit, soit par l'hydrure double de lithium et d'aluminium vers - 78 °C dans le tétrahydrofurane, soit par le borohydrure de sodium dans l'éthanol,
∗ pour obtenir l'amido-alcool trans de formule V : 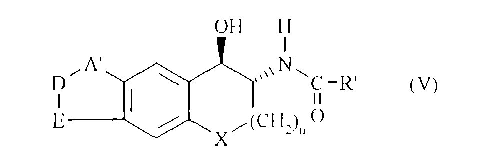 dans laquelle A'-D-E, X, n et R' ont les significations précédemment définies,
∗ qui est alors réduit à température ambiante au moyen d'hydrure double de lithium et d'aluminium dans le tétrahydrofurane
∗ pour obtenir un amino-alcool trans de formule VI : 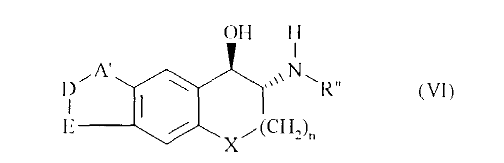 dans laquelle :
   A'-D-E, X et n ont les significations précédemment définies, et
   R" représente un groupement -CH₂-R' avec R' tel que défini précédemment,
∗ lequel composé de formule VI est traité par un halogénure d'acide de formule VII : 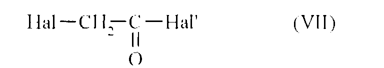 dans laquelle Hal et Hal', identiques ou différents, représentent chacun un atome de chlore ou de brome,
∗ pour obtenir un composé de formule VIII : 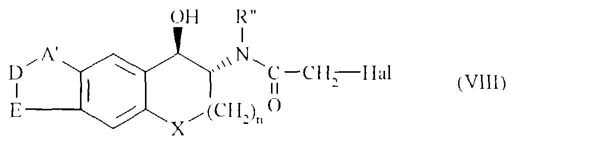 dans laquelle :
   A'-D-E, X, n et R'' ont les significations précédemment définies ;
∗ que l'on traite par un hydrure de métal alcalin, tel que par exemple l'hydrure de sodium,
∗ pour obtenir un composé de formule IX : 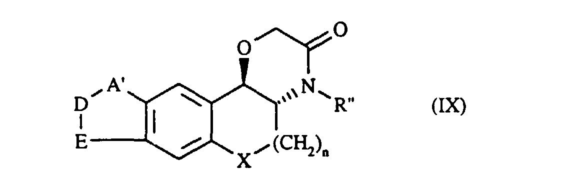 dans laquelle A'-D-E, X, n et R'' ont les significations prédécemment définies,
∗ lequel composé de formule IX est ensuite traité par le borane-sulfure de diméthyle
∗ pour donner un composé de formule X : 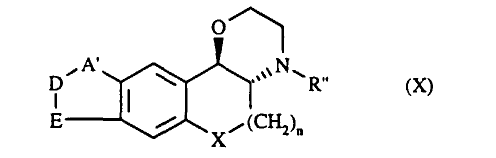 dans laquelle
   A'-D-E, X, n et R'' ont les significations précédemment définies,
∗ et dans le cas où R'' représente un radical benzyle, on débenzyle le dérivé correspondant de formule XI : 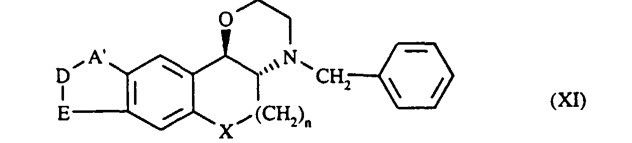 dans laquelle A'-D-E, X et n ont les significations précédemment définies,
∗ pour obtenir un composé de formule XII : 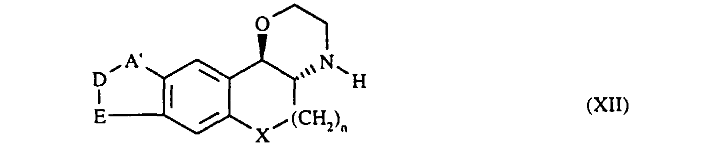 dans laquelle A'-D-E, X et n ont les significations précédemment définies, lequel est à son tour traité par un agent d'alkylation pour donner un composé de formule XIII : 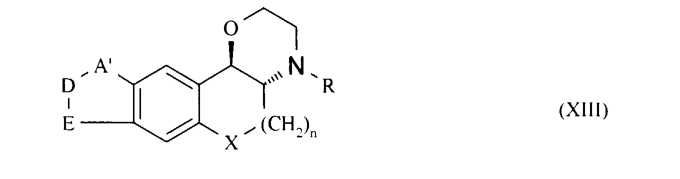 dans laquelle A'-D-E, X et n ont les significations précédemment définies, et R est tel que défini dans la formule (I) à l'exception de la valeur hydrogène,
∗ et l'on oxyde les composés de formule X, XII et XIII qui, réunis, forment l'ensemble des composés de formule I' : 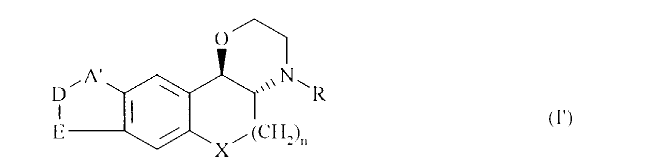 dans laquelle A'-D-E, X, n et R ont les significations précédemment définies, au moyen :
   - soit du réactif de Jones ou de 2,3-dichloro-5,6-dicyano-1,4-benzoquinone dans acide acétique et eau, dans le cas où -A'-D-E- prend les valeurs -(CH₂)₃- et -(CH₂)₄-,
   - soit de l'eau oxygénée ou l'acide métachloroperbenzoïque dans le cas où -A'-D-E- prend les valeurs -S-(CH₂)₂- et-S-CH=CH-,
∗ pour obtenir les composés de formule I" : 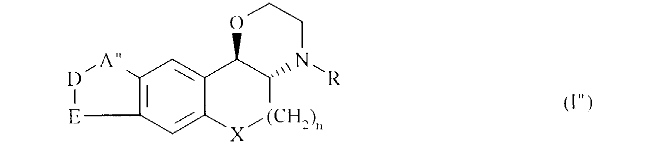 dans laquelle :
   X, n et R ont les significations précédemment définies et
   -A"-D-E- représente les groupes : 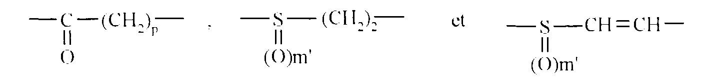 dans lesquels :
   p a la signification précédemment définie et m' représente 1 ou 2 ;
   et dans le cas où -A"-D-E- représente 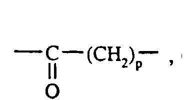 on réduit par le borohydrure de sodium ou par l'hydrure double de lithium et d'aluminium, les composés correspondants [c'est à dire les composés correspondant plus spécifiquement à la formule I''a : 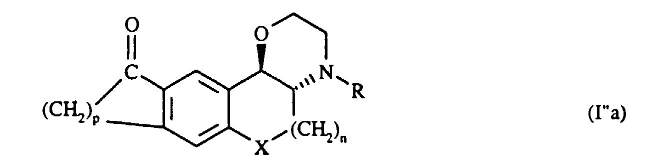
   dans laquelle p, X, n et R ont les significations précédemment définies]
∗ pour obtenir les composés de formule I''b : 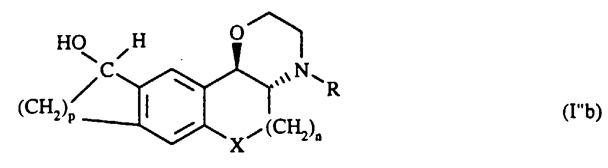 dans laquelle p, X, n et R ont les significations précédemment définies.

L'ensemble des composés de formule I' dans laquelle -A'-D-E- représente uniquement -S-(CH₂)₂ et -S-CH=CH- et des composés de formule I'' et I''b forme l'ensemble des composés de formule I.

Les formes optiquement actives des composés de formule I ont été obtenues soit à partir des formes optiquement actives des matières premières de formule II, soit par dédoublement des formes racémiques des composés de formule I, selon des méthodes connues de la littérature.

Les sels des composés de formule I avec des acides pharmaceutiquement acceptables ont été obtenus selon des méthodes classiques comme indiqué dans les exemples ci-après.

Les matières premières de formule II sont soit des produits connus soit des produits obtenus à partir de substances connues, selon des procédés connus, comme décrit ci-après dans les préparations 1 et 2.

La présente invention a également pour objet les compositions pharmaceutiques contenant comme principe actif un composé de formule I ou un de ses sels physiologiquement tolérable mélangé ou associé à un ou plusieurs excipients pharmaceutiques appropriés.

Les compositions pharmaceutiques ainsi obtenues se présentent généralement sous forme dosée renfermant de 0,5 à 25 mg de principe actif. Elles peuvent, par exemple, revêtir la forme de comprimés, dragées, gélules, suppositoires, solutions injectables ou buvables et être administrées par la voie orale, rectale ou parentérale selon les formes utilisées.

La posologie varie selon l'âge et le poids du patient, la voie d'administration et les traitements associés et s'échelonne de 0,5 à 25 mg de principe actif, 1 à 3 fois par jour.

Les exemples suivants, donnés à titre non limitatif, illustrent la présente invention.

Les points de fusion ont été déterminés soit à la platine chauffante de Kofler (K), soit à la platine chauffante sous microscope (MK).

### Synthèse des matières premières

Les matières premières utilisées dans les exemples suivants ont été préparées comme suit :

### Préparation 1 : Chlorhydrate du 3-amino 4-oxo 3,4-dihydro-2H-cyclopenta[g] benzopyrane

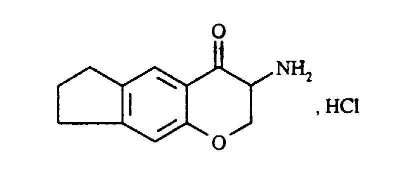

### Stade A : 3-(indan-5-yloxy)propionitrile

A température ambiante, on mélange 40,2 g d'indan-5-ol, 3 ml d'une solution de Triton B à 40 % dans le méthanol et 200 ml d'acrylonitrile fraîchement distillé. On porte à reflux pendant 48 heures, puis évapore au maximum l'acrylonitrile. Le résidu est repris à l'acétate d'éthyle, lavé à la soude N, à l'acide chlorhydrique N et avec une solution aqueuse saturée de chlorure de sodium. La phase organique est séchée sur sulfate de magnésium et concentrée, puis le résidu est recristallisé de l'éther isopropylique pour donner 40 g du composé désiré.
PF (K) : 66 °C, Rendement : 71 %.

### Stade B : Acide 3-(indan-5-yloxy)propionique

On porte à reflux pendant 4 heures, 20,9 g du composé précédent dans 33 ml d'acide sulfurique concentré et 44 ml d'eau. On ajoute alors 250 ml d'eau et refroidit dans un bain de glace. On filtre le solide formé, le rince à l'eau et le sèche sous vide, puis le recristallise de l'éther isopropylique pour obtenir 11,4 g du composé désiré.
PF (K) : 146°C, Rendement : 50 %.

### Stade C : 4 -oxo 3,4-dihydro-2H-cyclopenta[g]benzopyrane

A 14,8 g du composé précédent dans 145 ml de chloroforme à 0 °C, on ajoute 2 gouttes de diméthylformamide puis 9,4 ml de chlorure d'oxalyle goutte à goutte en 15 mn. On agite pendant 2 heures à température ambiante, puis ramène à 0 °C et ajoute par fractions en 20 minutes 14,3 g de chlorure d'aluminium. On laisse sous agitation la nuit à température ambiante, puis hydrolyse en versant dans 500 ml d'acide chlorhydrique 3N glacé et extrait au dichlorométhane. Les phases organiques sont lavées à l'acide chlorhydrique N, à la soude N et à l'eau, puis séchées sur sulfate de magnésium et concentrées. Le résidu est chromatographié sur silice (éluant : dichlorométhane) pour donner 12,4 g du composé désiré sous forme d'une huile.
Rendement: 81 %.

### Stade D : 4-hydroxyimino 3,4-dihydro-2H-cyclopental[g]benzopyrane

16,2 g du composé précédent, 25,1 g de chlorhydrate d'hydroxylamine et 25,1 g d'acétate de sodium dans 172 ml d'éthanol sont portés à reflux pendant 1 heure. On évapore le solvant, reprend au dichlorométhane et lave à l'eau. Après séchage sur sulfate de magnésium, évaporation, puis recristallisation de l'éthanol, on obtient 11,3 g du composé désiré.
PF (K) : 72-76 °C, Rendement : 65 %.

### Stade E : 4-p-toluènesulfonyloxyimino 3,4-dihydro-2H-cyclopenta[g]benzopyrane

A 11,2 g du composé précédent dans 55 ml de pyridine à 0 °C, on ajoute par fractions 12,6 g de chlorure de tosyle. On agite ensuite 3 heures à 0 °C, puis 20 heures à température ambiante. On verse alors dans 600 ml d'eau et extrait à l'éther. Les phases éthérées jointes sont lavées à l'eau, à l'acide sulfurique 0,5N et à l'eau, puis séchées sur sulfate de magnésium et concentrées pour donner 19,1 g du composé désiré.
PF (K) : 134 °C, Rendement : 97 %.

### Stade F : Produit titre

A de l'éthylate de sodium dans l'éthanol (préparé à partir de 1,4 g de sodium et 53 ml d'éthanol anhydre) à 0 °C, on ajoute 19 g du composé précédent dans 69 ml de benzène. Après 6 heures sous agitation à température ambiante, puis repos la nuit au réfrigérateur, on filtre le solide formé et le rince au benzène. Les filtrats sont versés sur 120 ml d'acide chlorhydrique 4N sous agitation énergique. Le solide formé est filtré et séché sous vide pour donner 8,55 g du composé désiré sous forme de chlorhydrate.
PF (K) > 260 °C, Rendement : 67 %.

### Préparation 2 : Chlorhydrate du 2,3,5,6,7,8-hexahydro-8-oxo-7-aminonaphto [2,3-b]thiophène

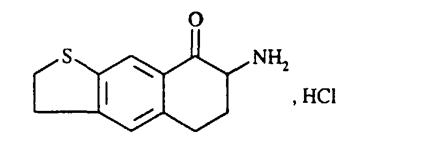

### Stade 1 : 2,3,5,6,7,8-hexahydro-8-hydroxyiminonaphto[2,3-b]thiophène

Dans un ballon bicol de 250 ml muni d'une agitation mécanique, 10,2 g (50 mmol) de 2,3,5,6,7,8-hexahydro-8-oxonaphto[2,3-b]thiophène (préparé selon W. Carruthers et coll. ; J. Chem. Soc., 1962, p 704-708), 14,6 g (210 mmol) de NH₂OH/HCl et 14,6 g (177 mmol) d'acétate de sodium sont mis en solution dans 100 ml d'éthanol et le mélange est porté à reflux une heure. Après retour à température ambiante, le mélange réactionnel est repris par 100 ml de CH₂Cl₂ puis filtré. Le filtrat est concentré. Le résidu est repris par 200 ml de CH₂Cl₂ et 100 ml d'eau. Les phases sont séparées, la phase organique est lavée quatre fois par 50 ml d'eau, séchée sur sulfate de magnésium et concentrée sous vide pour donner 9,96 g de produit attendu. Rendement: 91 %.

### Stade 2 : 2,3,5,6,7,8-hexahydro-8-p-toluènesulfonylhydroxyiminaphto[2,3-b]thiophène

Dans un ballon bicol de 250 ml muni d'une agitation mécanique et d'une entrée d'azote, 9,9 g (45 mmol) du produit obtenu au stade 1 sont mis en solution dans 55 ml de pyridine. La solution est refroidie à 0 °C et 12,9 g (68 mmol) de chlorure de tosyle sont additionnés par portion. Après 18 heures d'agitation à température ambiante, le mélange réactionnel est versé sur une solution aqueuse de NaHCO₃ (200 ml) à 0 °C. La phase aqueuse est extraite quatre fois par CH₂Cl₂. Les phases organiques sont rassemblées, lavées deux fois par HCI 1N, séchées sur sulfate de magnésium et concentrées sous vide pour donner 15,4 g de produit attendu. Rendement : 92 %.

### Stade 3 : produit titre

Dans un ballon tricol de 250 ml muni d'une agitation mécanique et d'une entrée d'azote, on ajoute, par portion, 0,58 g (25 mmol) de sodium à 21 ml d'éthanol sec. La solution d'éthylate de sodium ainsi obtenue est refroidie à 0-5 °C et 7,8 g (21 mmol) du produit obtenu au stade 2 en solution dans 54 ml de benzène sont ajoutés. Après 18 heures d'agitation à température ambiante, le mélange réactionnel est filtré et le filtrat est versé sur une solution aqueuse de HCI 4N (100 ml) à 0 °C. La solution ainsi obtenue est concentrée sous vide sans aller à sec et le solide brunâtre est filtré puis séché pour donner 3,3 g de produit attendu. Rendement : 62 %.

### Exemple 1 : Trans-3,4,4a,11b-tétrahydro 10-oxo 4-propyl 5H-cyclopenta[g] 1,4-oxazino[5,6-c] benzopyrane et son chlorhydrate

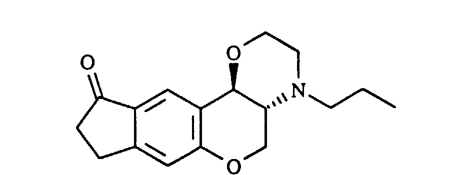

### Stade A : 3-propionylamino 4-oxo 3,4-dihydro-2H-cyclopenta[g]benzopyrane

A 8,45 g du composé obtenu à la préparation 1 en suspension dans 260 ml d'acétate d'éthyle à température ambiante, on ajoute 260 ml d'une solution aqueuse à 5 % de carbonate de sodium, puis 4,6 ml de chlorure de propionyle. On agite la nuit à température ambiante, sépare les phases et réextrait la phase aqueuse au dichlorométhane. Les phases organiques jointes sont séchées sur sulfate de magnésium et concentrées pour donner 8,55 g du composé désiré.
PF (K) : 159 °C, Rendement : 94 %.

### Stade B : 3-propionylamino 4-hydroxy 3,4-dihydro-2H-cyclopenta[g]benzopyrane

A 8,5 g du composé précédent dans 165 ml d'éthanol à température ambiante, on ajoute par fractions 1,3 g de borohydrure de sodium, maintient 24 heures sous agitation à température ambiante, puis évapore à sec. On reprend le résidu à l'eau et extrait au dichlorométhane. Les phases organiques sont séchées sur sulfate de magnésium et concentrées pour donner 8,4 g du composé désiré (cis/trans : 25/75). Rendement : 98 %.

### Stade C : trans-3-propylamino 4-hydroxy 3,4-dihydro-2H-cyclopenta[g]benzopyrane

A 3,02 g d'aluminohydrure de lithium dans 45 ml de tétrahydrofurane à température ambiante, on ajoute par fractions en 30 minutes 8,3 g du composé précédent en suspension dans 90 ml de tétrahydrofurane. On agite la nuit à température ambiante, puis 1 heure à reflux. On refroidit dans la glace et hydrolyse par 2,1 ml d'eau, puis, 1,67 ml de soude à 20 %, puis 7,6 ml d'eau, agite 3 heures à température ambiante, filtre les sels, les rince au tétrahydrofurane et concentre les filtrats. Le résidu ainsi obtenu est recristallisé 2 fois de l'acétate d'éthyle pour donner 3,6 g du composé désiré (100 % trans). PF (K) : 152 °C, Rendement : 46 %.

### Stade D: trans-3-(N-propyl N-chloroacétylamino) 4-hydroxy 3,4-dihydro-2H-cyclopenta[g]benzopyrane

A 3,5 g du composé précédent en suspension dans 210 ml d'acétate d'éthyle à température ambiante, on ajoute 210 ml d'une solution aqueuse à 5 % de carbonate de sodium, puis 1,67 ml de chlorure de chloroacétyle. On agite la nuit à température ambiante, sépare les phases et réextrait la phase aqueuse au dichlorométhane. Les phases organiques jointes sont séchées sur sulfate de magnésium et concentrées pour donner 4,6 g du composé désiré. Rendement quantitatif.

### Stade E : trans-3,4,4a,11b-tétrahydro 3-oxo 4-propyl 5H-cyclopenta[g] 1,4-oxazino[5,6-c]benzopyrane

A 0,84 g d'hydrure de sodium dans 25 ml de tétrahydrofurane à température ambiante, on ajoute goutte à goutte en 10 minutes 4,5 g du composé précédent solubilisé dans 70 ml de tétrahydrofurane et 20 ml d'acétonitrile. On agite à température ambiante pendant 24 heures, puis à 0 °C, détruit l'excès d'hydrure de sodium par addition de 9 ml de méthanol. On évapore à sec, reprend au dichlorométhane, lave à l'eau, sépare les phases et réextrait la phase aqueuse au dichlorométhane. Les phases organiques jointes sont séchées sur sulfate de magnésium et concentrées pour donner 3,9 g du composé désiré. PF (K) : 160 °C, Rendement : 97 %.

### Stade F : trans-3,4,4a,11b-tétrahydro 4-propyl 5H-cyclopenta[g] 1,4-oxazino[5,6-c]benzopyrane

A 3,8 g du composé précédent dans 150 ml de tétrahydrofurane à température ambiante, on ajoute goutte à goutte 12,5 ml de borane-sulfure de diméthyle. On porte à reflux la nuit, refroidit et solvolyse par 26 ml de méthanol, puis porte de nouveau 3 heures à reflux avant d'évaporer à sec. Le résidu ainsi obtenu est chromatographié sur silice (éluant : dichlorométhane/acétate d'éthyle 94/6) pour donner 2,58 g du composé désiré. PF (K) : 82 °C, Rendement : 79 %.

### Stade G : Produit titre

A 1,16 g du composé précédent solubilisé dans 150 ml d'acide acétique et 30 ml d'eau, on ajoute par fractions 2,86 g de 2,3-dichloro-5,6-dicyano-1,4-benzoquinone, puis porte à reflux 5 heures. Après évaporation à sec, on reprend au dichlorométhane et filtre sur célite. Après évaporation, le résidu est chromatographié sur silice (éluant : dichlorométhane/acétate d'éthyle) pour donner 0,37 g de base libre.
PF (MK) : 122-123 °C, après recristallisation de l'acétate d'éthyle.
Ce produit est repris dans un mélange éther/tétrahydrofurane, on y ajoute 1,1 équivalent d'éther chlorhydrique 3,7N puis on filtre le solide formé, le rince à l'éther et le sèche sous vide pour obtenir 0,38 g du produit titre sous forme de chlorhydrate. PF(MK) : 210-213 °C, Rendement : 28 %.

### Exemple 2 : Trans-3,4,4a,5,6,8,9,11b-octahydro-4-propyl-2H-thiéno[2,3-b]-1,4-oxazino[3,2-h]-naphtalène et son chlorhydrate

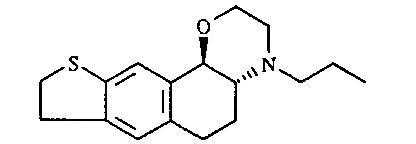

### Stade 1 : 2,3,5,6,7,8-hexahydro-8-oxo-7-propionylaminonaphto[2,3-b]thiophène

Dans un ballon tricol de 250 ml muni d'une agitation mécanique 2,56 g (10 mmol) du produit obtenu à la préparation 2 sont mis en suspension dans 74,5 ml d'acétate d'éthyle. 74,5 ml d'une solution aqueuse à 5 % de Na₂CO₃ sont additionnés à température ambiante et le mélange biphasique est vigoureusement agité jusqu'à dissolution totale. A cette solution biphasique 1,3 ml (15 mmol) de chlorure de propionyle sont additionnés à température ambiante. Après 5 heures d'agitation à température ambiante, les phases organique et aqueuse sont séparées et la phase aqueuse est extraite trois fois par de l'acétate d'éthyle. Les phases organiques sont rassemblées, séchées sur sulfate de magnésium et concentrées pour donner 2,47 g de produit attendu, Rendement : 90 %.

### Stade 2 : trans-2,3,5,6,7,8-hexahydro-8-hydroxy-7-propionylaminonaphto[2,3-b]thiophène

Dans un ballon tricol de 4 litres muni d'une agitation mécanique et d'une entrée d'azote 3,8 g (99,5 mmol) d'AlLiH₄ sont mis en suspension dans 850 ml de tétrahydrofurane sec. A -78 °C, 22,8 g (253 mmol) du produit obtenu au stade 1 en solution dans 1 060 ml de tétrahydrofurane sec sont lentement additionnés en maintenant la température du mélange réactionnel en dessous de - 70 °C. Après 2 heures d'agitation à - 78 °C, 2,5 ml d'une solution aqueuse saturée de NH₄Cl sont lentement additionnés et le mélange réactionnel est ramené à température ambiante. Le précipité est filtré et copieusement rincé par du tétrahydrofurane. Le filtrat est concentré sous vide. Le résidu (meringue de couleur sombre) est purifié par chromatographie sur silice (CH₂Cl₂/acétate d'éthyle : 90/10) pour donner 15 g de produit attendu. Rendement : 65 %

### Stade 3 : trans-2,3,5,6,7,8-hexahydro-8-hydroxy-7-(N-propylamino)naphto[2,3-b]thiophène

Dans un ballon tricol de 500 ml muni d'une agitation magnétique et d'une entrée d'azote, 2,3 g (61 mmol) d'AlLiH₄ sont mis en suspension dans 75 ml de tétrahydrofurane sec. 6,2 g (24,5 mmol) du produit obtenu au stade 2, en solution dans 97 ml de tétrahydrofurane sec sont lentement additionnés à température ambiante. Après 18 heures d'agitation à température ambiante, la réaction est stoppée par addition successive de 1,6 ml d'eau, 1,3 ml de NaOH à 20 % dans l'eau et enfin 5,8 ml d'eau. Le mélange réactionnel est agité 0,5 heure à température ambiante puis filtré. Le précipité est copieusement rincé par du tétrahydrofurane et le filtrat est concentré sous vide pour donner 1,37 g de produit attendu sous forme d'une meringue, Rendement : 97 %.

### Stade 4 : trans-2,3,5,6,7,8-hexahydro-8-hydroxy-7-(N-propyl N-chloroacétylamino)-naphto [2,3-b]thiophène

Dans un ballon tricol de 2 l muni d'une agitation mécanique, 7,37 g (28 mmol) du produit obtenu au stade 3 sont mis en solution dans 420 ml d'acétate d'éthyle. 420 ml d'une solution aqueuse à 5 % de Na₂CO₃ et 3,34 ml (42 mmol) du chlorure de l'acide chloroacétique sont successivement additionnés à température ambiante. Après 0,5 heure d'agitation à température ambiante, les phases organique et aqueuse sont séparées et la phase aqueuse est extraite 3 fois à l'acétate d'éthyle. Les phases organiques sont rassemblées, séchées sur sulfate de magnésium et concentrées. Le résidu est purifié par chromatographie sur silice (CH₂Cl₂/acétate d'éthyle : 90 /10) pour donner 6,69 g de produit attendu sous forme d'un solide, PF(K) : 162-164 °C, Rendement : 70 %.

### Stade 5 : trans-3,4,4a,5,6,8,9,11b-octahydro-3-oxo-4-propyl-2H-thiéno[2,3-b]-1,4-oxazino[3,2-h]naphtalène

Dans un ballon tricol de 500 ml muni d'une agitation magnétique et d'une entrée d'azote 2 g (49,3 mmol) de NaH (60 % dans l'huile, préalablement dégraissé par lavage au pentane) sont mis en suspension dans 37 ml de tétrahydrofurane sec. 6,96 g (19,7 mmol) du produit obtenu au stade 4 en solution dans 97 ml de tétrahydrofurane sec et 27 ml d'acétonitrile sec, sont lentement additionnés à température ambiante. Après 6 heures d'agitation à température ambiante, la réaction est stoppée par addition lente d'eau en refroidissant le mélange réactionnel avec un bain eau-glace. Le mélange réactionnel est repris par H₂O (200 ml) et CH₂Cl₂ (100 ml), les phases sont séparées et la phase aqueuse est extraite trois fois par CH₂Cl₂ (50 ml). Les phases organiques sont rassemblées, lavées par de la saumure, séchées sur sulfate de magnésium et concentrées pour donner 5,86 g de produit attendu sous forme d'un solide, PF (K) : 168-170 °C, Rendement : 98 %.

### Stade 6 : Produit titre

Dans un ballon bicol de 500 ml muni d'une agitation magnétique, d'un réfrigérant et d'une entrée d'azote, 4,83 g (15,9 mmol) du produit obtenu au stade 5 sont mis en solution dans 182 ml de tétrahydrofurane sec. 15,9 ml (15,9 mmol) de borane-sulfure de diméthyle [BH₃.S(CH₃)₂] sont lentement additionnés à température ambiante. Après 12 heures d'agitation à reflux, la réaction est stoppée par addition lente de méthanol en refroidissant le mélange réactionnel avec un bain eau-glace. Après 0,5 heure d'agitation à température ambiante, le mélange réactionnel est concentré sous vide. Le résidu est repris par 200 ml de méthanol et chauffé à reflux 1,5 heures en présence de 1 ml de HCI aqueux à 37 %, puis concentré sous vide. Le résidu solide est repris par 100 ml d'éther éthylique et 100 ml de soude 1N. La phase aqueuse est extraite quatre fois par 60 ml d'éther éthylique. Les phases organiques sont rassemblées, lavées par de la saumure, séchées sur sulfate de magnésium et concentrées pour donner 5,14 g d'une huile épaisse. Ce résidu est repris par 100 ml d'éther éthylique et le produit titre sous forme de chlorhydrate (4,67 g, Rendement : 90 %) est précipité par addition d'éther chlorhydrique.
PF(K) : > 260 °C.
RMN ¹H 200 MHz (CDCl₃/TMS), δ:
13,15 ppm(NH⁺, massif échangeable par D₂O) ; 7,3(s,1H) ; 6,9(s,1H) ; 5,25(d,1H) ; 4,65(td,1H) ; 4,15(dd,1H) ; 3,5(d,1H); 3,2-3,4(m,5H) ; 2,7-3,1(m,5H) ; 2,3-2,6(m,2H) ; 1,7-2,1(m,2H) ; 1,05(t,3H).

### Exemple 3 : Trans-3,4,4a,5,6,8,9,11b-octahydro-10-oxo-4-propyl-2H-thiéno[2,3-b]-1,4-oxazino[3,2-h]-naphtalène

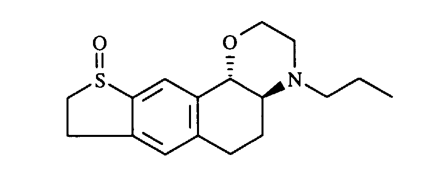

Dans un ballon bicol de 250 ml muni d'une agitation magnétique et d'un réfrigérant 3,56 g (10,9 mmol) du produit titre de l'exemple 2 sous forme de chlorhydrate sont mis en solution dans 77 ml d'eau. A cette solution sont successivement additionnés 5,5 ml d'HCl aqueux 1N et 1,56 ml (13,75 mmol) de H₂O₂ à 30%. Le mélange est chauffé à 80 °C et agité à cette température 0,5 heure. Après retour à température ambiante, le mélange réactionnel est alcalinisé par addition de soude 1N. La phase aqueuse est extraite trois fois par 50 ml d'éther éthylique. Les phases organiques sont rassemblées, lavées par de la saumure, séchées sur sulfate de magnésium et concentrées pour donner 3,57 g d'une huile épaisse. Ce résidu est purifié par chromatographie sur silice (éluant : CH₂Cl₂/méthanol ; 97/3) pour donner 1,09 g du sulfoxyde le moins polaire et 1,39 g du sulfoxyde le plus polaire. Une recristallisation de chacun des sulfoxydes dans respectivement 22 ml et 28 ml d'acétate d'éthyle permet d'obtenir 0,62 g du sulfoxyde le moins polaire et 0,87 g du sulfoxyde le plus polaire, soit un rendement global de 62 %.
*-Sulfoxyde le moins polaire :* PF(MK) : 170-171 °C.
RMN ¹H 400 MHz (DMSO d₆/TMS), δ : 7,75 (s,1H) ; 7,3(s,1H) ; 4,25(d,1H) ; 4,0-3,8(m,2H) ; 3,7-3,1(m,4H) ; 2,8(m,3H) ; 2,3(m,3H) ; 2,05(m,1H) ; 1,5(m,4H) ; 0,9(t,3H). *-Sulfoxyde le plus polaire :* PF(MK) : 169-170 °C.
RMN ¹H 200 MHz (DMSO d₆/TMS), δ : 7,75 (s,1H) ; 7,3(s,1H) ; 4,25(d,1H) ; 4,0-3,8(m,2H) ; 3,7-3,1(m,4H) ; 2,8(m,3H) ; 2,3(m,3H) ; 2,05(m,1H); 1,5(m,4H) ; 0,9(t,3H).

### Exemple 4 : Etude pharmacologique

La sélectivité pour les récepteurs D₃ vis à vis des récepteurs D₂ a été démontrée :
In vitro : par la technique du binding sur les récepteurs D₂ et D_{3.}
In vivo : par la capacité des produits de l'invention à moduler l'hypothermie induite chez le rat par l'agoniste dopaminergique D₃ : 7-OH-DPAT.

### 1. Matériel et méthode

### 1.1 In vitro-Binding

L'affinité des composés vis à vis des récepteurs D₃ et D₂ a été déterminée sur des préparations membranaires en utilisant le [¹²⁵I]-iodosulpride comme radioligand, le raclopride (10µM) déterminant la liaison non spécifique. Les résultats sont exprimés en IC₅₀.

### 1.2 In vivo - Hypothermie chez le rat

Les tests ont été réalisés sur des rats Wistar mâles de 200-250 g placés en cage individuelle avec libre accès à la nourriture et à l'eau. Les produits ont été solubilisés dans de l'eau distillée, à laquelle plusieurs gouttes d'acide lactique sont ajoutées. Les injections ont été effectuées dans un volume de 1,0 ml/kg par voie sous-cutanée. Les doses sont exprimées en terme de base. La température rectale des rats a été enregistrée en utilisant un thermistoprobe digital (Millan et al, J.P.E.T., 1993, 264, p 1364-1376). Dans un premier temps, les rats ont été injectés avec le composé à tester ou le véhicule, puis replacés dans leurs cages pendant 30 minutes. Puis, les rats ont subi une injection de 7-OH-DPAT (0,16 mg/kg) et ont été replacés dans leurs cages. Trente minutes plus tard, la température rectale a été mesurée et la différence a été déterminée par rapport aux valeurs basales (ΔT°C). La Dose Inhibitrice (95 % Limites de Confiance) pour réduire à 50 % l'effet du 7-OH-DPAT a été calculée selon la méthode de Finney (Statistical Method in Biological Assays, 2nd ed, Hafner Publishing, New York, 1964).

### 2. Résultats

### 2.1 Binding

Les affinités (IC₅₀) des produits de l'invention pour le récepteur D₃ sont comprises entre 10⁻⁹ M et 10⁻⁷ M, tandis que celles pour le récepteur D₂ sont comprises entre 10⁻⁷ M et 10⁻⁵ M.

### 2.2 Hypothermie chez le rat

L'effet des produits de l'invention au niveau du récepteur D₃ in vivo est illustré par le comportement du composé de l'exemple 2 dans le modèle de l'hypothermie. Les valeurs obtenues au cours de ce test sont rapportées dans le tableau suivant :

| **Injection 1** | **Injection 2** | **ΔT°C (a)** |
|---|---|---|
| Véhicule | Véhicule | 0,53 ± 0,23 |
| Véhicule | 7-OH-DPAT (0,16 mg/kg) | - 1,66 ± 0,07 |
| Produit de l'exemple 2 0,16 mg/kg | 7-OH-DPAT (0,16 mg/kg) | - 1,48 ± 0,44 |
| Produit de l'exemple 2 0,63 mg/kg | 7-OH-DPAT (0,16 mg/kg) | - 0,18* ± 0,21 |
| Produit de l'exemple 2 2,5 mg/kg | 7-OH-DPAT (0,16 mg/kg) | 0,56* ± 0,26 |

| | | |
|---|---|---|
| (a) les valeurs sont les moyennes ± S.E.M N≥5 par valeur | | |
| * p<0,05 versus véhicule/7-OH-DPAT selon le test de Dunnett | | |

La dose inhibitrice 50 (ID₅₀) (95 % LC = 95 % de Limites de Confiance) est de 0,41 (0,19-0,87) mg/kg, sc. Ceci met clairement en évidence que les produits de l'invention, non seulement reconnaissent in vitro le récepteur D₃ mais agissent, in vivo, par l'intermédiaire de ce même récepteur D₃.

## Revendications

1. Les composés tétracycliques de la 1,4-oxazine de formule I : 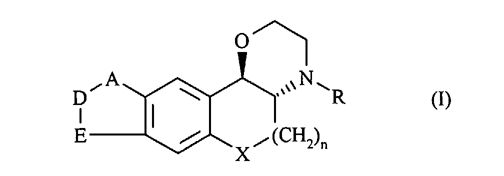 dans laquelle :
* **-A-D-E-** représente : 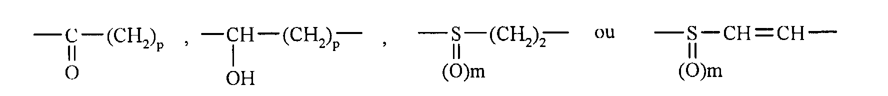 dans lesquels :
. p représente 2 ou 3 et
. m représente zéro, 1 ou 2 ;
* **X** représente :
. un groupe CH₂ et de plus,
. lorsque -A-D-E- représente 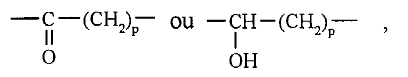 X peut aussi représenter un atome d'oxygène ;
* **n** représente :
. zéro ou 1 lorsque X représente le groupe CH₂, et
. uniquement 1 lorsque X représente un atome d'oxygène ;
* **R** représente :
. un atome d'hydrogène ou
. un radical (C₁-C₁₀)alkyle, (C₃-C₁₀)alcényle ou (C₃-C₁₀)alcynyle, chacun en chaîne droite ou ramifiée et chacun éventuellement substitué par un ou plusieurs radicaux cycloalkyles ayant de 3 à 8 atomes de carbone, ou par un radical aryle choisi parmi les radicaux phényle, thiényle et pyridyle chacun d'eux éventuellement substitué par un ou plusieurs substituants choisis parmi les atomes d'halogène, le radical hydroxy et les radicaux alkyle et alkoxy ayant chacun de 1 à 6 atomes de carbone en chaîne droite ou ramifiée;
lesquels composés possédant la jonction de cycle trans entre le cycle 1,4-oxazine et le cycle qui lui est adjacent,
sous forme racémique et d'isomères optiques,
ainsi que leurs sels d'addition avec un acide pharmaceutiquement acceptable.

2. Un composé de la revendication 1 qui est le *trans*-3,4,4a,5,6,8,9,11b-octahydro-4-propyl-2H-thiéno[2,3-b]-1,4-oxazino[3,2-h]-naphtalène et son chlorhydrate.

3. Le procédé de préparation des composés de la revendication 1 caractérisé en ce que
* l'on fait réagir :
. une amine primaire de formule II : 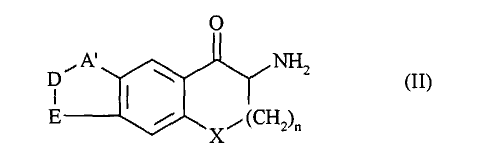 dans laquelle :
X et n ont les significations définies dans la revendication 1 et
-A'-D-E-représente : -(CH₂)₃-, -(CH₂)₄-, -S-(CH₂)₂- ou -S-CH=CH-
. avec un composé halogéné de formule III : 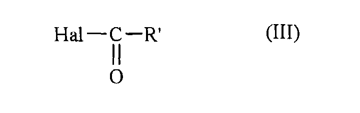 dans laquelle :
Hal représente un atome de chlore ou de brome et
R' représente :
un radical cycloalkyle ayant de 3 à 8 atomes de carbone ;
un radical aryle choisi parmi les radicaux phényle, thiényle et pyridyle chacun d'eux éventuellement substitué par un ou plusieurs substituants choisis parmi les atomes d'halogène, le radical hydroxy et les radicaux alkyle et alkoxy ayant chacun de 1 à 6 atomes de carbone en chaîne droite ou ramifiée, ou
un radical (C₁-C₉)alkyle, (C₂-C₉)alcényle ou (C₂-C₉)alcynyle, chacun en chaîne droite ou ramifiée et chacun éventuellement substitué par un ou plusieurs radicaux cycloalkyles ayant de 3 à 8 atomes de carbone, ou par un radical aryle choisi parmi les radicaux phényle, thiényle et pyridyle chacun d'eux éventuellement substitué par un ou plusieurs substituants choisis parmi les atomes d'halogène, le radical hydroxy et les radicaux alkyle et alkoxy ayant chacun de 1 à 6 atomes de carbone en chaîne droite ou ramifiée,
* pour obtenir un composé de formule IV : 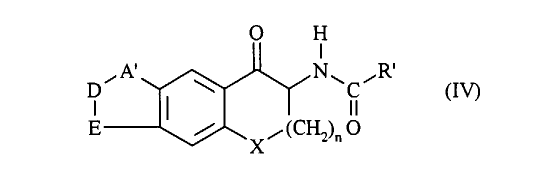 dans laquelle A'-D-E, X, n et R' ont les significations précédemment définies,
* lequel composé de formule IV est alors réduit, soit par l'hydrure double de lithium et d'aluminium vers - 78 °C dans le tétrahydrofurane, soit par le borohydrure de sodium dans l'éthanol,
* pour obtenir l'amido-alcool trans de formule V : 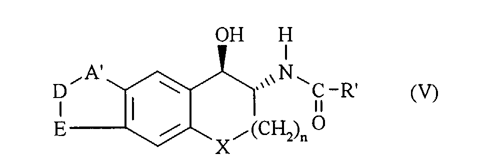 dans laquelle A'-D-E, X, n et R' ont les significations précédemment définies,
* qui est alors réduit à température ambiante au moyen d'hydrure double de lithium et d'aluminium dans le tétrahydrofurane
* pour obtenir un amino-alcool trans de formule VI : 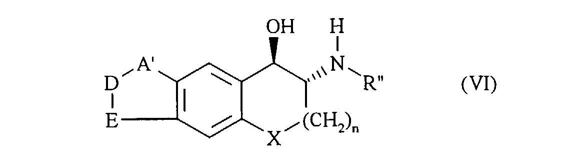 dans laquelle :
A'-D-E, X et n ont les significations précédemment définies, et
R" représente un groupement CH₂-R' avec R' tel que défini précédemment,
* lequel composé de formule VI est traité par un halogénure d'acide de formule VII : 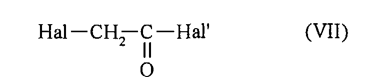 dans laquelle Hal et Hal', identiques ou différents, représentent chacun un atome de chlore ou de brome,
* pour obtenir un composé de formule VIII : 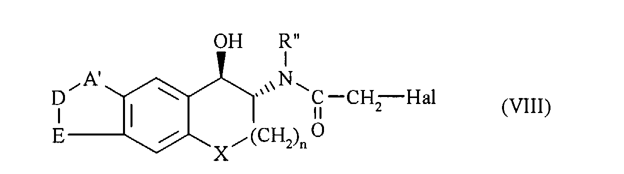 dans laquelle :
A'-D-E, X, n et R" ont les significations précédemment définies ;
* que l'on traite par un hydrure de métal alcalin,
* pour obtenir un composé de formule IX : 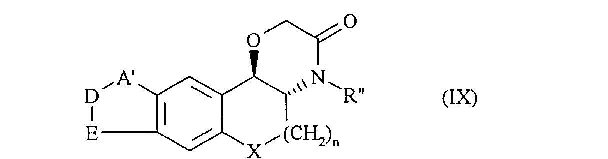 dans laquelle A'-D-E, X, n et R" ont les significations prédécemment définies,
* lequel composé de formule IX est ensuite traité par le borane-sulfure de diméthyle
* pour donner un composé de formule X : 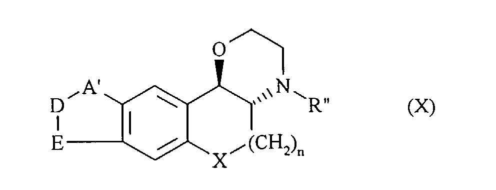 dans laquelle
A'-D-E, X, n et R" ont les significations précédemment définies,
* et dans le cas où R" représente un radical benzyle, on débenzyle le dérivé correspondant de formule XI : 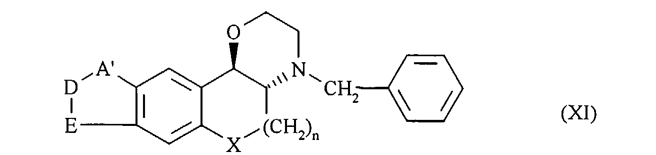 dans laquelle A'-D-E, X et n ont les significations précédemment définies,
* pour obtenir un composé de formule XII : 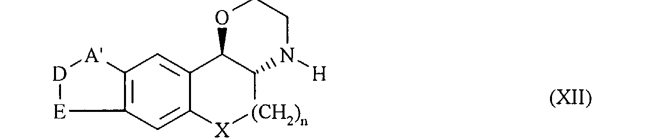 dans laquelle A'-D-E, X et n ont les significations précédemment définies,
lequel est à son tour traité par un agent d'alkylation pour donner un composé de formule XIII : 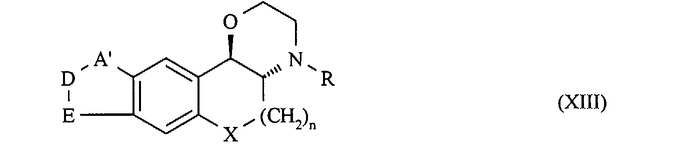 dans laquelle A'-D-E, X et n ont les significations précédemment définies, et R est tel que défini dans la formule (I) à l'exception de la valeur hydrogène,
* et l'on oxyde les composés de formule X, XII et XIII qui, réunis, forment l'ensemble des composés de formule I' : 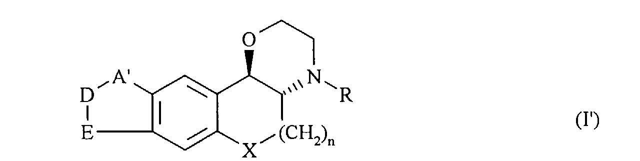 dans laquelle A'-D-E, X, n et R ont les significations précédemment définies, au moyen :
- soit du réactif de Jones ou de 2,3-dichloro-5,6-dicyano-1,4-benzoquinone dans acide acétique et eau, dans le cas où -A'-D-E- prend les valeurs -(CH₂)₃- et -(CH₂)₄-,
- soit de l'eau oxygénée ou l'acide métachloroperbenzoïque dans le cas où -A'-D-E- prend le valeurs -S-(CH₂)₂- et-S-CH=CH-,
* pour obtenir les composés de formule I": 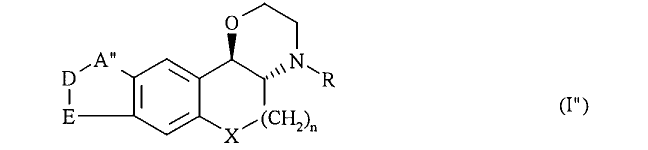 dans laquelle :
X, n et R ont les significations précédemment définies et
-A"-D-E-représente les groupes : 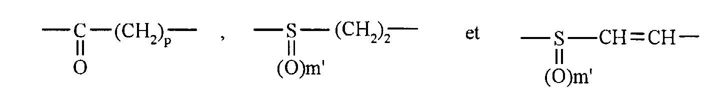
dans lesquels :
p a la signification précédemment définie et m' représente 1 ou 2 ;
et dans le cas où -A"-D-E- représente 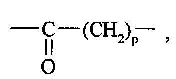 on réduit par le borohydrure de sodium ou par l'hydrure double de lithium et d'aluminium, les composés correspondants [c'est à dire les composés correspondant plus spécifiquement à la formule I"a : 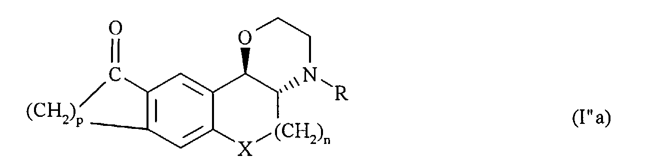 dans laquelle p, X, n et R ont les significations précédemment définies]
* pour obtenir les composés de formule I"b : 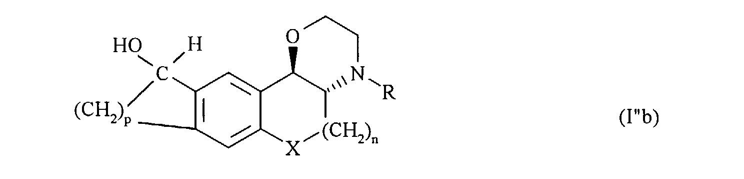 dans laquelle p, X, n et R ont les significations précédemment définies ;
l'ensemble des composés de formule I' dans laquelle -A'-D-E- représente uniquement -S-(CH₂)₂ et -S-CH=CH- et des composés de formule I" et I"b formant l'ensemble des composés de formule I ;
les formes optiquement actives des composés de formule I étant obtenues soit à partir des formes optiquement actives des matières premières de formule II, soit par dédoublement des formes racémiques des composés de formule I, et
si on le désire, les composés de formule I ainsi obtenus sont traités par des acides pharmaceutiquement acceptables pour obtenir les sels d'addition acides correspondants.

4. Les compositions pharmaceutiques utilisables dans le traitement de la maladie de Parkinson, des troubles de la mémoire, des troubles liés à l'abus de drogue, de la dépression et des états psychotiques, contenant comme principe actif un composé selon une des revendications 1 et 2, avec un ou plusieurs excipients pharmaceutiques appropriés.

## Claims

1. Tetracyclic 1,4-oxazine compounds of formula I: 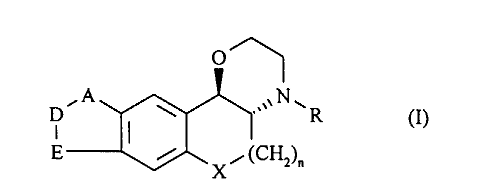 wherein :
* **-A-D-E-** represents : 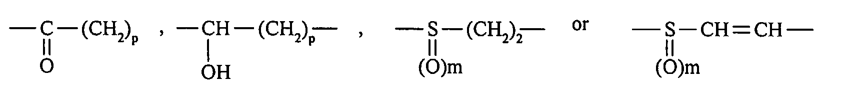 wherein :
. p represents 2 or 3 and
. m represents zero, 1 or 2 ;
* **X** represents :
. a CH₂ group and in addition,
. when -A-D-E- represents 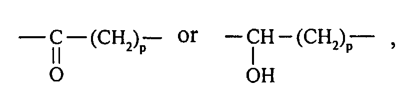 X may also represent an oxygen atom ;
* **n** represents :
. zero or 1 when X represents a CH₂ group and
. solely 1 when X represents an oxygen atom ;
* **R** represents :
. a hydrogen atom or
. a (C₁-C₁₀)alkyl, (C₃-C₁₀)alkenyl or (C₃-C₁₀)alkynyl radical, each in straight or branched chain and each optionally substituted by one or more cycloalkyl radicals having from 3 to 8 carbon atoms, or by an aryl radical selected from the radicals phenyl, thienyl and pyridyl, each of which is optionally substituted by one or more substituents selected from halogen atoms, the hydroxy radical, and alkyl and alkoxy radicals each having from 1 to 6 carbon atoms in straight or branched chain;
which compounds have a *trans* ring junction between the 1,4-oxazine ring and the ring adjacent thereto,
in racemic form or in the form of optical isomers,
and also their addition salts with a pharmaceutically acceptable acid.

2. A compound of claim 1 which is *trans*-3,4,4a,5,6,8,9,11b-octahydro-4-propyl-2H-thieno[2,3-b]-1,4-oxazino[3,2-h]naphthalene and its hydrochloride.

3. A process for the preparation of the compounds of claim 1, characterised in that
*
. a primary amine of formula II: 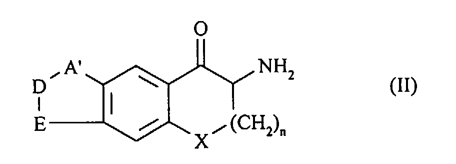 wherein:
X and n are as defined in claim 1 and
-A'-D-E- represents : -(CH₂)₃-, -(CH₂)₄-, -S-(CH₂)₂- or -S-CH=CH-
. is reacted with a halogenated compound of formula III: 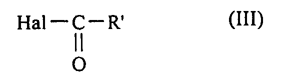
wherein :
Hal represents a chlorine or bromine atom and
R' represents :
a cycloalkyl radical having from 3 to 8 carbon atoms ;
an aryl radical selected from the radicals phenyl, thienyl and pyridyl, each of which is optionally substituted by one or more substituents selected from halogen atoms, the hydroxy radical, and alkyl and alkoxy radicals each having from 1 to 6 carbon atoms in straight or branched chain, or
a (C₁-C₉)alkyl, (C₂-C₉)alkenyl or (C₂-C₉)alkynyl radical, each in straight or branched chain and each optionally substituted by one or more cycloalkyl radicals having from 3 to 8 carbon atoms, or by an aryl radical selected from the radicals phenyl, thienyl and pyridyl, each of which is optionally substituted by one or more substituents selected from halogen atoms, the hydroxy radical, and alkyl and alkoxy radicals each having from 1 to 6 carbon atoms in straight or branched chain,
* to obtain a compound of formula IV : 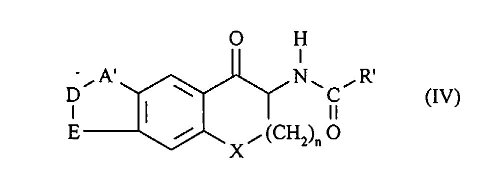 wherein A'-D-E, X, n and R' are as defined hereinbefore,
* which compound of formula IV is then reduced, either with lithium aluminium hydride at approximately -78°C in tetrahydrofuran, or with sodium borohydride in ethanol,
* to obtain the *trans*-amido-alcohol of formula V : 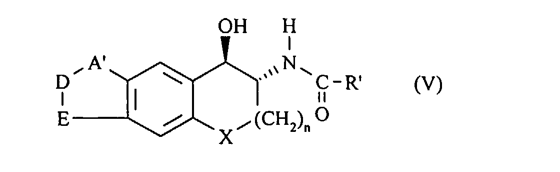 wherein A'-D-E, X , n and R' are as defined hereinbefore,
* which is then reduced at room temperature by means of lithium aluminium hydride in tetrahydrofuran
* to obtain a *trans*-amino-alcohol of formula VI : 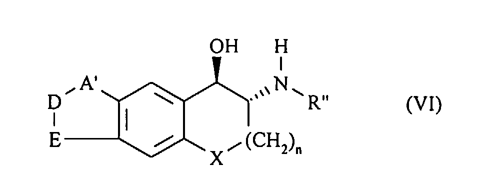 wherein :
A'-D-E, X and n are as defined hereinbefore, and
R" represents a CH₂-R' group wherein R' is as defined hereinbefore;
* which compound of formula VI is treated with an acid halide of formula VII: 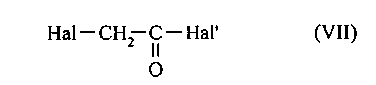 wherein Hal and Hal', which are identical or different, each represents a chlorine or bromine atom,
* to obtain a compound of formula VIII : 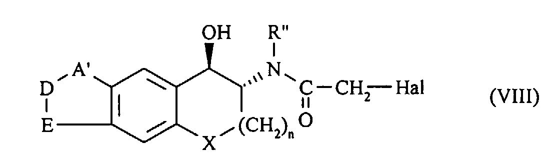 wherein :
A'-D-E, X, n and R" are as defined hereinbefore
* which is treated with an alkali metal hydride,
* to obtain a compound of formula IX : 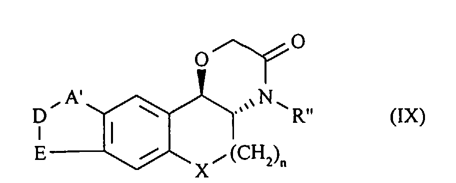 wherein A'-D-E, X, n and R" are as defined hereinbefore,
* which compound of formula IX is then treated with borane-dimethyl sulphide
* to yield a compound of formula X : 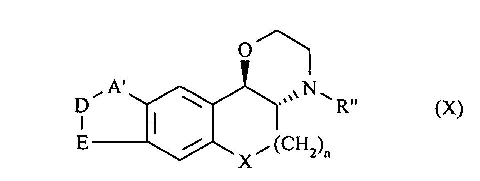 wherein
A'-D-E, X, n and R" are as defined hereinbefore,
* and, when R" represents a benzyl radical, the corresponding compound of formula XI: 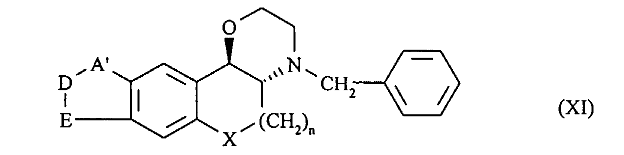 wherein A'-D-E, X and n are as defined hereinbefore is debenzylated
* to obtain a compound of formula XII: 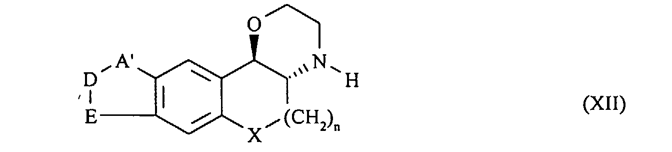 wherein A'-D-E, X and n are as defined hereinbefore,
which in turn is treated with an alkylation agent to yield a compound of formula XIII : 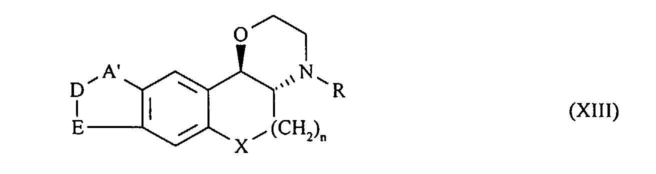 wherein A'-D-E, X and n are as defined hereinbefore, and R is as defined for formula (I) with the exception of the meaning hydrogen,
* and the compounds of formula X, XII and XIII which, taken together, form the totality of the compounds of formula I': 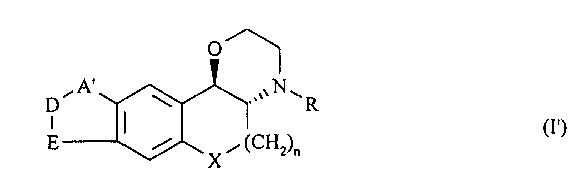 wherein A'-D-E, X, n and R are as defined hereinbefore, are oxidised by means :
- either of Jones reagent or of 2,3-dichloro-5,6-dicyano-1,4-benzoquinone in acetic acid and water, in the case where -A'-D-E- represents -(CH₂)₃- or -(CH₂)₄-,
- or of hydrogen peroxide or meta-chloroperbenzoic acid in the case where -A'-D-E-represents -S-(CH₂)₂- or -S-CH=CH-,
* to obtain the compounds of formula I" : 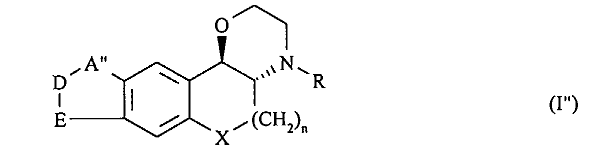 wherein:
X, n and R are as defined hereinbefore and
-A"-D-E- represents the group : 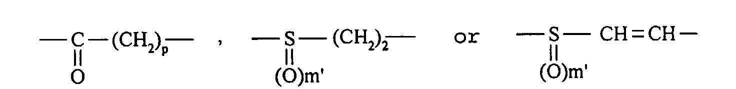
wherein :
p is as defined hereinbefore and m' represents 1 or 2 ;
and, in the case where -A"-D-E- represents 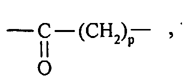 the corresponding compounds [that is to say the compounds corresponding more specifically to formula I"a : 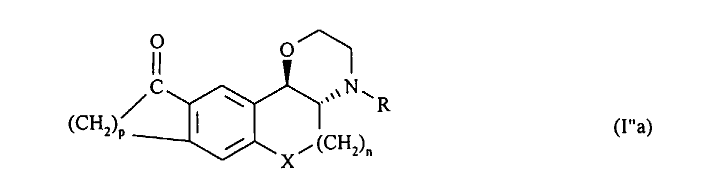 wherein p, X, n and R are as defined hereinbefore] are reduced with sodium borohydride or with lithium aluminium hydride
* to obtain the compounds of formula I"b : 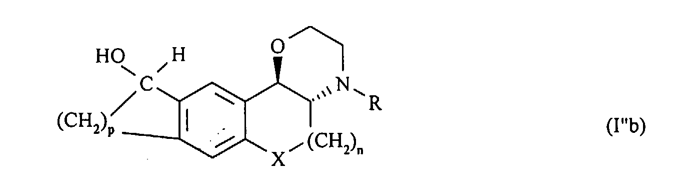 wherein p, X, n and R are as defined hereinbefore;
the totality of the compounds of formula I' wherein -A'-D-E- represents solely -S-(CH₂)₂ or -S-CH=CH- and of the compounds of formula I" and I"b forming the totality of the compounds of formula I;
the optically active forms of the compounds of formula I being obtained either from the optically active forms of the starting materials of formula II, or by splitting the racemic forms of the compounds of formula I, and
if desired, the compounds of formula I thus obtained are treated with pharmaceutically acceptable acids to obtain the corresponding acid addition salts.

4. Pharmaceutical compositions that can be used in the treatment of Parkinson's disease, of memory disorders, of disorders associated with drug abuse, of depression and of psychotic states, comprising as active ingredient a compound according to either claim 1 or claim 2 together with one or more appropriate pharmaceutical excipients.

## Patentansprüche

1. Tetracyclische 1,4-Oxazin-Verbindungen der Formel I: 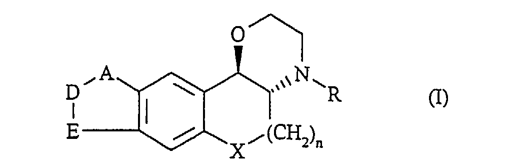 in der:
* **-A-D-E-**: 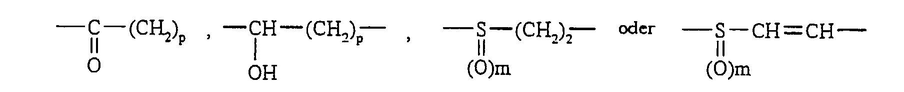 in denen:
. p 2 oder 3 und
. m Null, 1 oder 2 darstellen, bedeuten;
* **X:**
. eine CH₂-Gruppe bedeutet und,
. wenn -A-D-E- 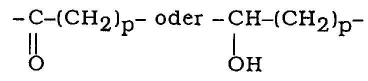 darstellt, X auch ein Sauerstoffatom bedeuten kann;
* **n**:
. Null oder 1, wenn X die CH₂-Gruppe darstellt, und
. lediglich 1, wenn X ein Sauerstoffatom darstellt, bedeutet;
* **R:**
. ein Wasserstoffatom oder
. ein (C₁-C₁₀)-Alkyl-, (C₃-C₁₀)-Alkenyl- oder (C₃-C₁₀)-Alkinylrest, jeweils mit gerader oder verzweigter Kette und die jeweils gegebenenfalls substituiert sein können durch einen oder mehrere Cycloalkylreste mit 3 bis 8 Kohlenstoffatomen oder einen Arylrest ausgewählt aus Phenyl-, Thienyl- und Pyridylresten, die ihrerseits jeweils gegebenenfalls durch einen oder mehrere Substituenten ausgewählt aus Halogenatomen, Hydroxygruppen und Alkyl- und Alkoxyresten mit 1 bis 6 Kohlenstoffatomen in gerader oder verzweigter Kette substituiert sein können, bedeutet;
welche Verbindungen die trans-Ringbindung zwischen dem 1,4-Oxazinring und dem benachbarten Ring aufweisen,
in racemischer Form oder in Form der optischen Isomeren,
sowie deren Additionssalze mit einer pharmazeutisch annehmbaren Säure.

2. Verbindung nach Anspruch 1, nämlich *trans*-3,4,4a,5,6,8,9,11b-Octahydro-4-propyl-2H-thieno[2,3-b]-1,4-oxazino[3,2-h]-naphthalin und dessen Hydrochlorid.

3. Verfahren zur Herstellung der Verbindungen nach Anspruch 1, dadurch gekennzeichnet, daß
* man
. ein primäres Amin der Formel II: 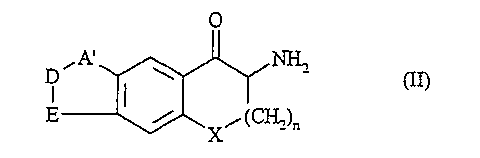 in der:
X und n die in Anspruch 1 angegebenen Bedeutungen besitzen und
-A'-D-E-: -(CH₂)₃, -(CH₂)₄-, -S-(CH₂)₂- oder -S-CH=CH- bedeutet
. mit einer Halogenverbindung der Formel III umsetzt: 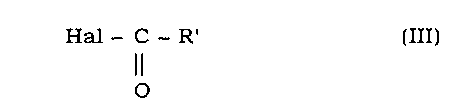 in der:
Hal ein Chlor- oder Bromatom und
R':
einen Cycloalkylrest mit 3 bis 8 Kohlenstoffatomen;
einen Arylrest ausgewählt aus Phenyl-, Thienyl- und Pyridylresten, die jeweils gegebenenfalls durch einen oder mehrere Substituenten ausgewählt aus Halogenatomen, Hydroxygruppen und Alkyl- und Alkoxyresten mit jeweils 1 bis 6 Kohlenstoffatomen in gerader oder verzweigter Kette substituiert sein können, oder
einen (C₁-C₉)-Alkyl-, (C₂-C₉)-Alkenyl- oder (C₂-C₉)-Alkinylrest, jeweils mit gerader oder verzweigter Kette und die jeweils gegebenenfalls substituiert sein können durch einen oder mehrere Cycloalkylreste mit 3 bis 8 Kohlenstoffatomen oder einen Arylrest ausgewählt aus Phenyl-, Thienyl-und Pyridylresten, die Jeweils ihrerseits gegebenenfalls durch einen oder mehrere Substituenten ausgewählt aus Halogenatomen, Hydroxygruppen und Alkyl- und Alkoxyresten mit jeweils 1 bis 6 Kohlenstoffatomen in gerader oder verzweigter Kette substituiert sein können,
* zur Bildung einer Verbindung der Formel IV: 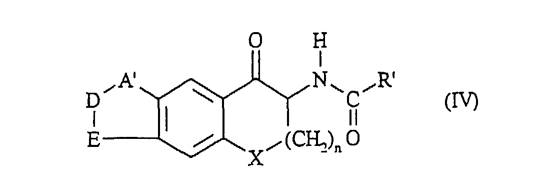 in der A'-D-E, X, n und R' die oben angegebenen Bedeutungen besitzen,
* welche Verbindung der Formel IV anschließend entweder mit Lithiumaluminiumhydrid bei etwa -78°C in Tetrahydrofuran oder mit Natriumborhydrid in Ethanol reduziert wird,
* zur Bildung des trans-Amido-alkohols der Formel V: 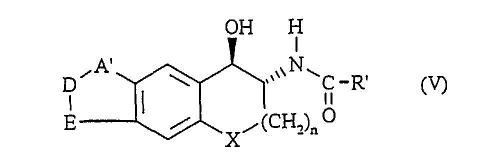 in der A'-D-E, X, n und R' die oben angegebenen Bedeutungen besitzen,
* welcher anschließend bei Raumtemperatur mit Lithiumaluminiumhydrid in Tetrahydrofuran reduziert wird,
* zur Bildung eines trans-Amino-alkohols der Formel VI: 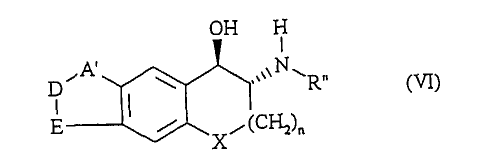 in der:
A'-D-E, X und n die oben angegebenen Bedeutungen besitzen und
R" eine Gruppe CH₂-R' darstellt, worin R' die oben angegebenen Bedeutungen besitzt,
* welche Verbindung der Formel VI mit einem Säurehalogenid der Formel VII: 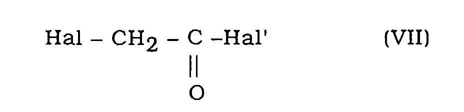 in der Hal und Hal', die gleichartig oder verschieden sein können, jeweils ein Chloratom oder ein Bromatom bedeuten,
* zur Bildung einer Verbindung der Formel VIII: 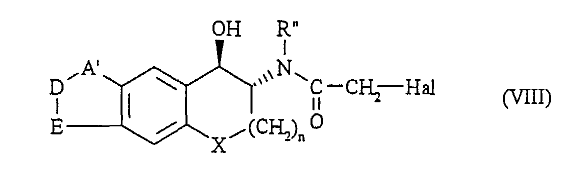 in der:
A'-D-E, X, n und R" die oben angegebenen Bedeutungen besitzen;
* welche man mit einem Alkalimetallhydrid behandelt,
* zur Bildung einer Verbindung der Formel IX: 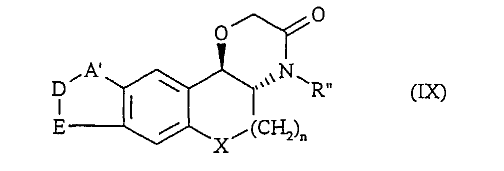 in der A'-D-E, X, n und R" die oben angegebenen Bedeutungen besitzen,
* welche Verbindung der Formel IX anschließend mit Boran-Dimethylsulfid behandelt wird
* zur Bildung einer Verbindung der Formel X: 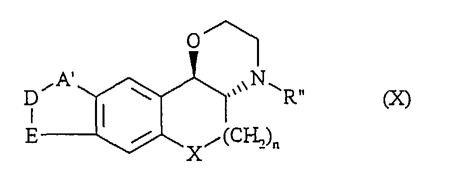 in der
A'-D-E, X, n und R" die oben angegebenen Bedeutungen besitzen,
* und in dem Fall, da R" eine Benzylgruppe bedeutet, man das entsprechende Derivat der Formel XI: 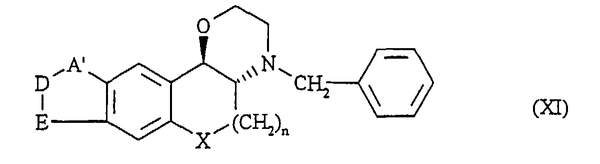 in der A'-D-E, X und n die oben angegebenen Bedeutungen besitzen, debenzyliert,
* zur Bildung einer Verbindung der Formel XII: 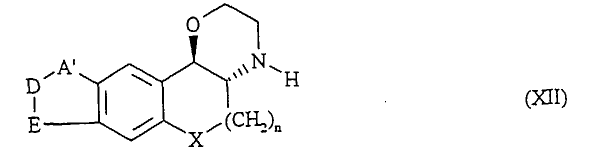 in der A'-D-E, X und n die oben angegebenen Bedeutungen besitzen, welche ihrerseits mit einem Alkylierungsmittel behandelt wird zur Bildung einer Verbindung der Formel XIII: 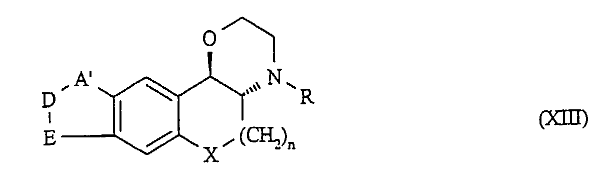 in der A'-D-E, X und n die oben angegebenen Bedeutungen besitzen und R die bezüglich der Formel (I) angegebenen Bedeutungen mit Ausnahme der Bedeutung Wasserstoff besitzt,
* und man die Verbindungen der Formeln X, XII und XIII, welche die Gesamtheit der Verbindungen der Formel I' bilden: 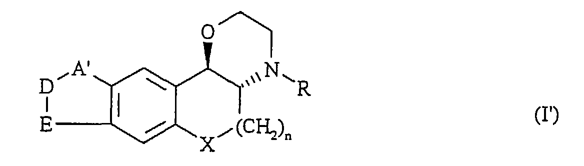 in der A'-D-E, X, n und R die oben angegebenen Bedeutungen besitzen, mit Hilfe:
- entweder des Jones-Reagens oder von 2,3-Dichlor-5,6-dicyano-1,4-benzochinon in Essigsäure und Wasser in dem Fall, da -A'-D-E- die Bedeutungen -(CH₂)₃- und -(CH₂)₄- besitzt,
- oder von Wasserstoffperoxid oder m-Chlorperbenzoesäure in dem Fall, da -A'-D-E- die Bedeutungen -S-(CH₂)₂- und -S-CH=CH- aufweist, oxidiert,
* zur Bildung der Verbindungen der Formel I": 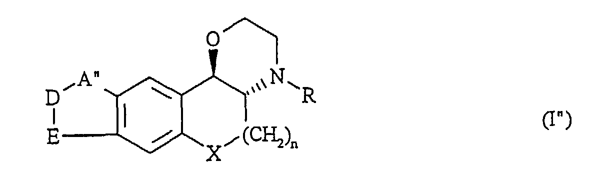 in der:
X, n und R die oben angegebenen Bedeutungen besitzen und
-A"-D-E- die Bedeutungen der folgenden Gruppen besitzt: 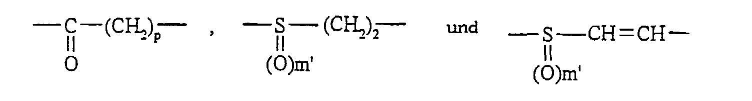
in denen:
p die oben angegebenen Bedeutungen besitzt und m' 1 oder 2 darstellt;
und man in dem Fall, da -A"-D-E- die Bedeutung 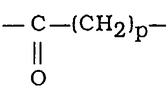 besitzt, man die entsprechenden Verbindungen [das heißt die Verbindungen, die spezifisch der Formel I"a entsprechen: 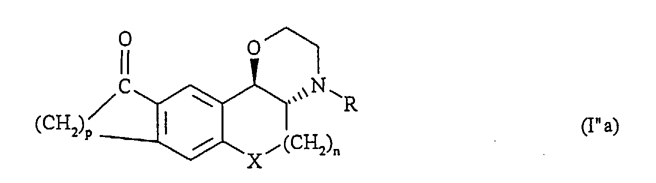 in der p, X, n und R die oben angegebenen Bedeutungen besitzen]
mit Natriumborhydrid oder mit Lithiumaluminiumhydrid reduziert,
* zur Bildung der Verbindungen der Formel I"b: 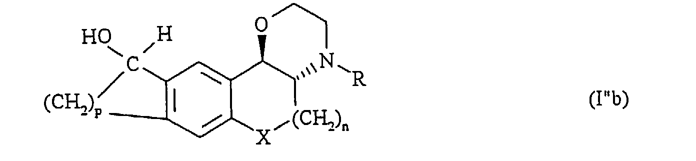 in der p, X, n und R die oben angegebenen Bedeutungen besitzen;
wobei die Gesamtheit der Verbindungen der Formel I', in der -A'-D-E- lediglich -S-(CH₂)₂) und -S-CH=CH- bedeutet, und die Verbindungen der Formel I" und I"b die Gesamtheit der Verbindungen der Formel I darstellen;
wobei man die optisch aktiven Formen der Verbindungen der Formel I entweder ausgehend von optisch aktiven Formen der Ausgangsmaterialien der Formel II oder durch Aufspaltung der racemischen Formen der Verbindungen der Formel I herstellt, und
man gewünschtenfalls die in dieser Weise erhaltenen Verbindungen der Formel I mit pharmazeutisch annehmbaren Säuren behandelt zur Bildung der entsprechenden Säureadditionssalze.

4. Pharmazeutische Zubereitungen für die Behandlung der Parkinsonschen Krankheit, von Gedächtnisstörungen, von Störungen, die mit dem Drogenmißbrauch verknüpft sind, von Depressionen und psychotischen Zuständen, enthaltend als Wirkstoff eine Verbindung nach einem der Ansprüche 1 und 2 zusammen mit einem oder mehreren geeigneten pharmazeutischen Trägermaterialien.
